# EUROPEAN PATENT SPECIFICATION

(11) **EP 3 612 145 B1**
(45) Date of publication and mention of the grant of the patent: **02.11.2022**
(21) Application number: 18723613.8
(22) Date of filing: 18.04.2018
(51) Int. Cl.: A61F 13/53, A61F 13/49, A61F 13/02, A61L 15/28, A61L 15/60, C08L 5/04, B01J 20/24

(54) **BIOBASED SUPER-ABSORBING POLYMERS**
BIOBASIERTE SUPERABSORBIERENDE POLYMERE
POLYMÈRES SUPERABSORBANTS D'ORIGINE BIOLOGIQUE

(30) Priority: 20.04.2017 NL 2018754
(43) Date of publication of application: 26.02.2020
(73) Proprietor: Technische Universiteit Delft, 2628 CN Delft (NL)
(72) Inventor: CARUS, Edmund, 2600 AA Delft (NL); PICKEN, Stephen James, 2600 AA Delft (NL)
(74) Representative: Vogels, Leonard Johan Paul
(86) International application number: PCT/NL2018/050242
(87) International publication number: WO 2018/194451

(56) References cited:
- WO-A1-94/17227
- WO-A1-03/090801
- WO-A1-2015/190927
- WO-A1-2016/087795
- WO-A2-99/20318
- CN-A- 104 532 401
- GB-A- 2 332 629
- US-A- 5 256 477
- US-A1- 2005 137 272
- US-A1- 2013 069 000
- POURJAVADI ET AL: "MBA-crosslinked Na-Alg/CMC as a smart full-polysaccharide superabsorbent hydrogels", CARBOHYDRATE POLYM, APPLIED SCIENCE PUBLISHERS, LTD. BARKING, GB, vol. 66, no. 3, 2 November 2006 (2006-11-02), pages 386-395, XP005715396, ISSN: 0144-8617, DOI: 10.1016/J.CARBPOL.2006.03.013
- HOEFER DIRK ET AL: "Biotechnologically produced microbial alginate dressings show enhanced gel forming capacity compared to commercial alginate dressings of marine origin", JOURNAL OF MATERIALS SCIENCE: MATERIALS IN MEDICINE, SPRINGER NEW YORK LLC, UNITED STATES, vol. 26, no. 4, 19 March 2015 (2015-03-19) , pages 1-9, XP035491103, ISSN: 0957-4530, DOI: 10.1007/S10856-015-5492-5 [retrieved on 2015-03-19]

## Description

### FIELD OF THE INVENTION

The present invention is in the field of extraction of a product for liquid absorption comprising a low-density biopolymer, such as that obtainable from a granular sludge, in particular bacterial alginate, wherein the biobased polymer acts as a super-absorbing material, the product itself, and a method of obtaining said product.

### BACKGROUND OF THE INVENTION

In various fields of technology super-absorbent polymers are used, such as agriculture, personal hygiene products and in medical products.

Until around 1980, the commonly used absorbent materials were cellulose or fibre-based products used in either woven items, opened fibre formats such as absorbent cotton (cotton wool) and fluff pulp (bleached kraft or thermomechanical) or as seen in tissue. The water absorbent capacity of these types of materials is only in the region of 11 times their weight, but most of it is lost under low to moderate pressure. At that time initial attempts were made to use super absorbent polymers (SAPs) into sanitary napkins and diapers. The SAP content increased to over 30% in absorbent fluff-pulp cores by the end of the decade. Current SAP quantities in the "core" of infant diapers and adult incontinence products can now be up to 100% in "fluff-less" products thus allowing for thinner products with excellent "fit" and low obtrusiveness. SAPs have now become integral in the design of absorbent cores, these being attained by advances in fluff-pulp technology by air laying technologies for example coupled in many cases by new thinking in adhesives. In an example SAP is deposited from reservoirs in a printing roll onto a substrate on a grid. Thermoplastic adhesive is used to form a fibrous network applied to the SAP to immobilize this in both the dry and wet states. The system involves chemical bonding and curled cellulose fibres. In an alternative for the continuous production of a flexible liquid-absorbing material having two outer layers, between which are inserted at least two stretchable-elastic intermediate layers, a matrix is created able to gather the absorbent material. In a further alternative bonding beads are used to bond two layers, the first featuring an array of "absorbent receiving pockets" into which SAP is inserted. A second layer is bonded to this using non water soluble bead between the pockets to impart core integrity. The TWE Core is part of an absorbent structure comprising a nonwoven substrate having a void volume suitable to be penetrated with SAP particles and adhesives. SAP is dispersed according to a size distribution gradient along the depth direction of the core. Also a sandwich web comprising a first and second outer layer may be provided, preferably comprising cellulose fibres. Superabsorbent material is sandwiched between the outer layers and individualized fibres adapted to be interspaced between the SAP are provided.

In a further approach round-shaped particles with micropores comprising SAPs have been introduced recently, having an innovative liquid distribution mechanism, making it a highly efficient superabsorbent polymer in e.g. a diaper core.

SAPs in fibre form have also been developed, which can be precisely tailored into a diverse range of nonwoven fabrics, tapes and spun yarn formats. Ease of downstream handling and processing can result in exceptionally substantive and uniform distribution profiles within the final product constructions. As such, they may offer truly unparalleled levels of consistent absorption performance and media integrity, though costs are still a concern.

In the early 1960s work was conducted on materials to improve water conservation in soils. A resin based on the grafting of acrylonitrile polymer onto the backbone of starch molecules was provided. The hydrolysed product gave water absorption greater than 400 times its weight of water. A wide range of grafting combinations were attempted including work with acrylic acid, acrylamide and polyvinyl alcohol (PVA). Chemical alternatives relate to cellulose derived materials, such as carboxy methyl cellulose (CMC), acrylic acid, polyvinyl alcohol (PVA) and isobutylene maleic anhydride (IMA). Application of these polymers into products ran into processing problems however. Also natural products, such as sphagnum moss, were considered in this respect.

The agricultural industry is the fastest-growing market sector for SAPs. Their use is now an established water conservation technique and is widely practiced in different parts of the world. The growing population and increased demand for food in developing nations are the key drivers for the demand for the SAP market in these agriculture applications. The important use areas can very broadly be categorized into food production, gardening, horticulture, forestry, also and to a lesser extent in lawns, and in recreational areas such as play grounds, and golf-courses. Over time, many technologically improved SAPs used in soil amendments applications have been based on cross-linked acrylic-acrylamide co-polymers with potassium neutralization. Though toxic intermediates are sometimes used in the manufacturer of these polymers, cross-linked SAPs used for soil amendments are non-toxic, inert with increased shelf life and last for years in the ground. Initial SAPs had low swell capacity, prevented oxygen from passing to plant roots, and had high cost per unit water held. The application of SAPs directly to soil can result in earlier seed germination and/or blooming, decreased irrigation requirements, increased propagation, increased crop growth, increased crop production, and decreased soil crusting. SAP amended soils have better nutrient release, high nitrification, reduced micro flora and bacterial content. In addition benefits from SAP usage include improved soil drainage, also, reduced pesticide use, improved fertilizer usage, and reduced irrigation watering by 30% to 50% as well as reduced labour costs.

It is noted that synthetic copolymer SAPs show poor performance in agriculture because they are typically composed of small, fine particles having an 80 mesh size. One inherent limitation of fine-mesh particles is that they cannot be used in typical granule applicators which require particle sizes of at least 25 mesh. Further, the SAP films and powders cannot be applied with granular fertilizers, granular pesticides, or other granular agricultural additives. Research is in hand to address this area of low particle size.

A drawback with SAPs is that they are mostly based on petroleum, such as cross-linked polyacrylic acid. The SAPs are used in large amounts (3.5 Mtons per year). Various natural materials, such as cellulose, starch, chitin, and natural gums, have been used as the basis to form the main polymer chains, much research being published. It is noted that SAPs made without starch are referred to as "synthetic copolymers." These studies have, for the most part, not yet been able to match either the cost or performance of the oil-based SAPs, leading numerous companies to explore possible routes to produce bio-acrylic acid and in particular precursors thereof, such as by (genetically modified) microbes. In an alternative more bio-based approach a starch basis is used obtained from agricultural feedstock, but it is not clear yet of the obtained SAP meets requirements.

Medical products are considered to have resulted as a follow-on from the above advances. Therein a multitude of references to hydrogels are given, but hydrogels are regarded as a different area of technology in view of the SAPs way of performance.

So despite great advances being made in SAPs and their incorporation into hygiene products, the whole area of the sustainability of SAPs still needs to be further addressed.

Recently it has been found that biobased polymeric substances, such as extracellular polymeric substances, in particular linear polysaccharides, obtainable from granular sludge can be produced in large quantities. These substances relate to biobased carboxylic acid, which may be present in an ionic form (e.g. cationic or anionic). Examples of such production methods can be found in WO2015/057067 A1, and WO2015/050449 A1, whereas examples of extraction methods for obtaining said biobased polymers can be found in Dutch Patent application NL2016441 and in WO2015/190927 A1. Specific examples of obtaining these substances, such as aerobic granular sludge and anammox granular sludge, and the processes used for obtaining them are known from Water Re-search, 2007, doi:10.1016/j.waters.2007.03.044 (anammox granular sludge) and Water Science and Technology, 2007, 55(8-9), 75-81 (aerobic granular sludge). Further, Li et al. in "Characterization of alginate-like exopolysaccharides isolated from aerobic granular sludge in pilot plant", Water Research, Elsevier, Amsterdam, NL, Vol. 44, No. 11 (June 1 2010), pp. 3355-3364) recites specific alginates in relatively raw form. Details of the biopolymers can also be found in these documents, as well as in Dutch Patent applications NL2011609, NL2011542, NL2011852, NL2017470, and NL2012089. Also reference can be made to the Nereda^{®} process. These documents, and there contents, such as characteristics (e.g. molecular weights, dynamic viscosity, shear rate, tensile strength, and flexural strength) of the biobased polymers, are incorporated by reference.

Advantageously, granules of granular sludge can be readily removed from a reactor by e.g. physical separation, settling, centrifugation, cyclonic separation, decantation, filtration, or sieving to provide extracellular polymeric substances in a small volume. Compared to separating material from a liquid phase of the reactor this means that neither huge volumes of organic nor other solvents (for extraction), nor large amounts of energy (to evaporate the liquid) are required for isolation of the extracellular polymeric substances.

Extracellular polymeric substances obtainable from granular sludge (preferably obtained from granular sludge) do not require further purification or treatment to be used for some applications, hence can be applied directly. When the extracellular polymeric substances are obtained from granular sludge the extracellular polymeric substances are preferably isolated from bacteria (cells) and/or other non-extracellular polymeric substances. The granular sludge can be suitably produced by bacteria belonging to the order Pseudomonadaceae, such as pseudomonas and/or Acetobacter bacteria (aerobic granular sludge); or, by bacteria belonging to the order Planctomycetales (anammox granular sludge), such as Brocadia anammoxidans, Kuenenia stuttgartiensis or Brocadia fulgida; or, by algae, such as brown algae.

Extraction of biopolymers from aerobic granular sludge is an emerging topic, and so far amongst others extraction with alkaline substances, such as Na₂CO₃, NaOH or NaOCl, and likewise with compounds having two amine groups, have been suggested and implemented. The processes provide acceptable results for some further applications, but still produce a mixture of components present and need relatively harsh conditions. Na₂CO₃ is found to provide biopolymers with an average number molecular weight of 50-75 kDa, NaOH at a pH of 11-12 provides biopolymers with an average number molecular weight of 20-30 kDa, and NaOCl provides biopolymers with an average number molecular weight of about 120 kDa(100-150 kDa).

However, for various applications the extracellular polymeric substances, in this document also referred to as "biopolymers" or "biobased carboxylic acids", can not be used directly, e.g. in view of insufficient purity, a typical (brownish) colouring of the extracellular polymeric substances, etc.

With the term "microbial process" here a microbiological conversion is meant.

Some applications of ionic biopolymeric substances per se or in extracted form have been considered. For instance application of the polymers in paper as a sizing agent, and application on a concrete or metal surface have been found beneficial. Further uses and applications, however, are still limited in extent. From another perspective use and application of biobased substances instead of e.g. chemically based substances is nowadays considered an advantage, especially in view of sustainability. Hence there is a need for further fully biobased applications, methods and products.

Algae alginate (e.g. from seaweed) has found application in various products, especially as gelling agents. Calcium alginate wound dressings are used worldwide in view of creating a moist wound environment that encourages more effective healing. Alginate is found to have various advantages such as meeting toxicological standards for medical devices (ISO 10993), being manufactured in accordance with ISO 13485, it forms gels on contact with sodium ions, and it is capable of undergoing ion exchange with other metal ions. They are used in dressings for many types of wet wounds, which promotes healing and reduces pain. Also antibacterial properties may be provided, such as by addition of silver. Thereto alginate is typically incorporated into a fabric, especially in view of prolonged bactericidal action. A fabric can be provided in various forms, such as roll form, and it may be sterilized and packaged.

Practical application of biopolymers such as alginate has been limited however.

Some further documents recite isolation of alginate from aerobic granular sludge, such as Li (see above) and WO2015/190927 A1. The methods described therein have certain disadvantages making the obtained alginate less suited for further use.

Some documents recite absorbent materials with high absorbency, of which some are biobased or biodegradable. Pourjavadi in Carbohydrate Polymers, Applied Science Pub. Vol. 66, no. 3, p. 386-395 (2-11-2006), WO 94/17227 A1, US2005/137272 A1, WO 99/20318 A2), WO 2016/087795 A1, GB 2 332 629 A, US 5,256,477 A, US2013/069000 A1, CN 104 532 401 A, and WO 03/090801 A1 (D10

The present invention relates to a product comprising biodegradable material, such as the above alginate, which material has been upgraded by further processing and thereby making it available for a range of further applications, which overcomes one or more of the above disadvantages, without jeopardizing functionality and advantages.

### SUMMARY OF THE INVENTION

A further aspect of the present invention relates to a method of obtaining a super-absorbing biobased polymer material, as defined in claims 12-14. Recently it has been found that extraction with non-solvents produce low-density biopolymers such as fibrous polymer, granules, foam-like polymer, and fibre-foam-like polymer. The biopolymer may be alginate, such a bacterial alginate. The super-absorbing biobased polymer may be a non-woven material, the super-absorbing biobased polymer may comprise fibres, and the super-absorbing biobased polymer may comprise a powder with particle sizes > 0.1 mm. This low-density biopolymer is now topic of further research and practical applications. The density of the biopolymer is < 0.5 gr/cm³, preferably < 0.25 gr/cm³, more preferably < 0.1 gr/cm³, such as < 0.075 gr/cm³ for fibres, < 0.2 gr/cm³ for granules, and preferably < 0.02 gr/cm³ for foam. It is noted that none of the above mentioned documents recite such low density biopolymers and it is rather unexpected to obtain such low density polymers. For an exemplary Na-ALE an exopolymer in these granules is swollen, and may be crosslinked with Ca²⁺ or other similar means. One to ten times lower densities are achievable. The foam may have a regular structure, such as a honey-comb structure. In particular it has been found that the present biopolymer has super-absorbing characteristics for liquids. It can therefore be used in products which require such super-absorbing characteristics, such as a diaper, an incontinence product, a personal hygiene product, a soil-improver, a water retainer, a wound dressing, especially for burn wounds, a thermal insulator, a thermal clothing layer, a thermal insulating panel, concrete, a protective sheet or laminate, such as a cable wrap or tape, a liquid absorbents, and an absorbent pan component. The present superabsorbent polymers (SAPs) and products comprising said SAPs swell upon uptake of water and other fluids without dissolving. It is noted that typical application of SAPs (before and during use) expose the material to mechanical forces/stresses, which may cause movement, shape change, change in particle location/spatial distribution, which is undesirable for optimal functioning of the SAP for its purpose. The inventors have unexpectedly found that biopolymers such as alginate/ALE during recovery from solution via precipitation, using a non-solvent (especially acetone, and likewise other mono- or dialkyl ketones, alkoxy alkanols, such as iso propoxy ethanol) may give rise to a fibrous, non-woven morphology which is found particularly beneficial to enhance a mechanical stability of the alginate precipitate when used as a SAP. Prior art methods may form non-woven materials and the like, but these methods require further steps, including forming fibres, cutting fibres, making a non-woven material, and hence are more costly and less efficient. The fibres typically have a diameter of at least 1 up to 100 micrometre and a length of 0.1-5 mm. They can be converted into a material having a non-woven mesh size, for instance where branching and/or crosslinking points are positioned at a distance between 1-2000 µm. It is found that a distance of less than 1 micron provides too fast absorption and too fragile structures, and a large chance of blocking of pores, whereas a distance of more than 2 mm results in a mesh of the non-woven material that has too much air and is not useful as a SAP. The SAP as a non-woven material may require further processing thereof, such as chemical/physical crosslinking of polymer chains such as to allow swelling without dissolving, and to form a swollen hydrogel. The fibre diameter can be optimized such as to achieve rapid swelling. It is noted that a level of cross-linking may influence fluid uptake capacity (with and without application of external force/stress). The SAP material may be further modified, such as to provide a core shell morphology to prevent fibre adhesion and gel blocking of the superabsorbing structure. For application in a product subsequent processing of the precipitated fibrous SAP non-woven material may involve actions as pressing, cutting, needling, gluing, embedding in a textile or non-woven garment, attaching in place using stitching and similar methods. It is noted that benefits of a fibrous morphology in general are known and considered desirable. The current practice may involve fibre spinning and subsequent weaving, felting, needling, punching etc. to form a desired superabsorbing element. It is considered that these process steps however are complex, time consuming and/or expensive compared to the present invention which provides at least an alternative thereto. In addition a desired morphology of the present SAPs can be influenced by boundary conditions of the inventor's method of precipitation, concentration of biopolymer and non-solvent, flow rates, solvent/non-solvent ratio(s), and method of agitation (as described in the above mentioned patent documents of the present inventors). The present SAPs can be considered an economic commodity starting point for new and totally sustainable SAPs, which can be obtained at low costs.

The present superabsorbent material can be based on 10 wt. % to 99.8 wt.%, preferably 25 wt.% to 99 wt.%, and more preferably 30 wt.% to 90 wt.% of biobased SAP, 0 wt.% to 10 wt.%, preferably 0.01 wt.% to 7 wt.%, and more preferably 0.05 wt.% to 5 wt.% of one or more cross- linkers, 0 wt.% to 30 wt.%, preferably 1 wt.% to 20 wt.%, and more preferably 5 wt.% to 10 wt.% of water-soluble polymers; 0-2 wt.% buffer, preferably 0.1 wt.% to 1 wt.%, and more preferably 0.2 wt.% to 0.5 wt.%, 0-2 wt.% additive, such as a plasticizer, a filler, preferably 0.1 wt.% to 1 wt.%, and more preferably 0.2 wt.% to 0.5 wt.%, 0-5 wt.% lipids, preferably 0.5 wt.% to 3 wt.%, and more preferably 1 wt.% to 2 wt.%, wherein the sum of the component weights amounts to 100 wt.%. Water-soluble polymers are preferably biobased as well, such as starches or starch derivatives, and polyglycols, which can be polymerized into the superabsorbent polymer particles. The water-soluble polymers can also serve as graft basis for monomers to be polymerized.

In a specific exemplary embodiment SAPs incorporated into tapes provide a rapid performance during the critical first minute of response to water. The SAP can be laminated and evenly distributed between various nonwoven materials so that all the SAP contributes efficiently to the water blocking properties of the tape. Water swellable tapes are provided in insulative, semi-conductive, laminated and marine versions to suit a specific cable application.

In a specific exemplary embodiment of a sheet for packaging for meat, fish and poultry SAPs absorb escaping liquids and store these for an unlimited time. The food can thus be transported and stored hygienically. It stays fresh longer and does not have to be repacked so often. The present SAPs are considered food safe and non-toxic, although this will depend on the specific extraction process in view of the source of the material.

In a specific exemplary embodiment the present SAPs may be used in industrial applications for absorbing aqueous solutions. In these industrial settings the SAPs are used in a variety of applications from solidifying water in the drilling and mining industry to the construction industry including oil fracking. The polymers can be used in any application where aqueous solidification and spill management is of concern.

In a specific exemplary embodiment the present SAPs may be used in concrete technology, such as an internal curing agent in high-strength concrete, for improving concrete characteristics, such as viscosity, frost resistance, water regulation, etc.

In an exemplary embodiment the present SAPs may be used in bandages, gauzes and other dressing materials as they can mitigate higher levels of blood loss while remaining effective as doctors and nurses operate or attend to wounds in other ways. Superabsorbency also is found to reduce an amount of unconfined bodily fluids, curtailing chances of disease transferral and even infection. A recent application relates to dressings where a superabsorbent component in the dressing acts to "plug" wounds literally thus providing extra crucial life saving time before more detailed medical treatment. Superabsorbent dressings have an extra fluid-handling capacity. They may be designed to be used on wounds of varying types such as those that produce moderate to high volumes of wound fluid (known as exudate). In such cases it has been shown that after only 3 days, dressing change frequency was reduced from once daily to twice weekly in 80% of patients. The superabsorbent dressing seemed to reduce complications associated with exudate production, stimulate wound healing, and increase patient comfort; it may also save time and costs for caregivers. If the wound has fluctuating volumes of exudate (i.e. low to high), or a heterogeneous healing process (mixed wound bed), a superabsorbent dressing with a built-in atraumatic surface contact layer may be more suitable. Moisture management is found important, both to maintain a moist wound healing environment and to protect the periwound skin from maceration. Superabsorbents are found to vary in a way they absorb and retain fluid and how they function under compression. Some superabsorbents can lock fluid inside the dressing. This fluid may contain bacteria and proteases, which can be harmful to the wound and surrounding skin (periwound area). Due to their enhanced fluid-handling capacity and absorbency, superabsorbent dressings are designed for longer wear times and to reduce maceration. Superabsorbent dressings are suggested as being particularly useful for wound cleansing. Superabsorbent dressings may have multiple layers, which can include a wound contact layer, an inner core containing fibres, powders, crystals or gelling agents to increase fluid absorption and retention properties, and some have a fluid repellent backing layer to prevent strikethrough. Some superabsorbent dressings absorb via osmosis, others through a capillary action while retaining a moist wound interface. Non-adherent superabsorbent dressings require a secondary bandage, while others have adhesive borders to keep them in place. Superabsorbents can be used for drug delivery, such as transdermal delivery, spill capture, dental pads, bed mats (disposable and washable, and for urine capture-bottles and bed-pans. Superabsorbents can be used on a variety of wounds, including pressure ulcers, venous ulcers, diabetic foot ulcers, arterial and neuropathic ulcers, post-operative wounds, traumatic wounds, first and second-degree burns, oncology wounds, and donor sites. The present low-density SAPs are found to improve several of the above aspects.

The process specifically relates to extracting an ionic biopolymer, such as an anionic biopolymer, and specifically bacterial alginate (ALE) from an aqueous solution. In a step an aqueous mono-valent anionic biopolymer such as bacterial alginate (ALE) solution is provided, wherein the alginate is present in an amount of 0.1-30 wt.%. Thereafter 20-80 vol.%, preferably 30-60 vol. %, more preferably 35-55 vol.%, such as 40-50 vol.%, non-solvent, such as mono- or dialkyl ketones, such as acetone, and alkoxy alkanols, such as iso propoxy ethanol, alkylene amines, such as alkylene mono-, di- or tri-amines, such as acetone (dimethyl ketone) is added under mixing, such as by stirring or moderate shaking, to the solution. The acetone may typically have a water content of 5-25 %v/v. It is preferred to use relatively dry acetone having a water content as low as possible, e.g. 1-5 %v/v water/acetone mixture. The mixing is preferably a vigorous mixing, e.g. using an impellor at relatively high speeds (600-1000 rpm). Surprisingly thereafter the biopolymer can be extracted from the solution with a relatively high yield. Throughout the description weight percentages are relative to a total mass of the solution, unless indicated otherwise; likewise volume percentages are calculated. The steps may be performed at an elevated temperature (e.g. 310-375 K), or at an (close to) environmental temperature (e.g. 280-300 K). The present process is typically carried out in a reactor.

A step in the extraction process is treatment of the alginate solution to be able to extract the polymer as a solid material. It is found that of a range of possible extraction solvents acetone (dimethyl ketone) is by far the best in terms of subsequent sample removal via filtering and solvent evaporation to obtain a monovalent (e.g. sodium) salt of the extracted polymer. In an example the addition of about 50% (45-55%) acetone to a 3% sodium alginate solution in water yields about 80% extraction efficiency via filtration of the formed fibrous mass of precipitated Na-alg/Na-ALE. The precipitate is not sticky/gel like contrary to the more commonly used precipitation with ethanol (known from current alginate extraction processes applied to seaweed harvesting and by analogy is intended to be used for Nereda^{®} sludge). No doubt other (non)solvents could in principle be used, but surprisingly the results thereof were found to be relatively poor: ethanol forms a sticky precipitate, which is difficult to work up; ether, such as diethyl ether, does not form a precipitate, and it does not mix with water; butanol forms a two phase system, with a gel like consistency; DMSO (dimethyl sulfoxide) lets alginate precipitate, but it forms a sticky gel, and DMSO is not volatile hence can not be separated easily from the alginate; ethylene glycol shows no precipitation and forms a homogenous gel; and methanol shows a similar behaviour to ethanol, and is in fact even more sticky. The acetone extraction also works extremely well for other monovalent anionic biopolymers such as ammonium alginate solutions and for drying of gel-like acid ALE. Acetone is considered to be a rather optimal treatment method as it is easy to work-up again via distillation/membrane separation and analogous methods; also it is a naturally occurring compound so it does not provide any major issue if trace amounts remain in the product. Part of the acetone recovery can be combined with the drying of the biopolymer precipitate, which may involve heat such as with distillation. Other processes are available to get rid of the remaining acetone/water supernatant.

The obtained biopolymers resemble those of the prior art, but are different in certain aspects, such as characterized in the claims; i.e. compared to e.g. algae alginate chemical and physical characteristics are found to be different, such as a lipid content is much higher (2-5 wt.%) and the polymers have a fibrous structure comparable to cotton wool. Typical fibres obtained (before further treatment) are a few (1-2) mm to a few (1-5) cm long having a thickness of 10-250 µm and fibres may have a yellowish appearance. The different characteristics result in different applications of the present biopolymers now being possible, or likewise being impossible, compared to those of the prior art.

The product for liquid absorption comprises a super-absorbing material of biobased polymer, which biobased polymer comprises 50-99.8 wt.% low-density polymer. Surprisingly the present low-density polymer acts as a SAP. As it is biobased it is easy degradable. The super-absorbing material may comprise further compounds, such as fillers, cross-linking agent or residues thereof, a buffer, further additives as plasticizers, and lipids. A remainder is typically water and optionally trace residues from pre-processing steps such as non-solvent. The present product provides excellent absorbing properties, typically >20 (volume up-taken liquid/volume SAP), and most often > 30. In addition it is stable over time, it is capable of absorbing many different (types of) liquids, it remains it physical and chemical properties, is can withstand typical boundary conditions upon use, such as shear, etc.

Thereby the present invention provides a solution to one or more of the above mentioned problems. Advantages of the present invention are detailed throughout the description.

### DETAILED DESCRIPTION OF THE INVENTION

The present invention relates in a first aspect to a product according to claim 1.

In an exemplary embodiment of the present product the ionic biobased polymer is produced by bacteria, such as alginate, preferably ALE.

In an exemplary embodiment of the present product the super-absorbing biobased polymer is a non-woven material, wherein the super-absorbing biobased polymer is immobilized in the product, such as by at least one of pressing, needling, gluing, embedding in a textile or non-woven garment, attaching, stitching, weaving, felting, needling, and punching. The present biobased polymer can be incorporated in various ways into a further or final product, which makes it versatile.

In an exemplary embodiment of the present product the super-absorbing biobased polymer is treated by at least one of cross-linking, such as with multivalent ions, such as Ca²⁺, Fe²⁺, Al³⁺, Mg²⁺, and Fe³⁺, with nanoparticles, such as nano-CaCO₃, and graphite, by radiation branching, by chemical modification, such as for preventing dissolving of the fibre, for preventing adhesion, by physical modification, such as changing a fibre cross-section and/or fibre length, by surface modification, such as changing hydrophobicity and hydrophilicity, and by providing a shell. By further treatment characteristics of the present low-density polymers can be adapted and changed to further specifications and/or requirements.

In an exemplary embodiment of the present product the super-absorbing biobased polymer fibre have a diameter of 1-100 µm, a length of 0.1-5 mm, and a mesh of 1-2000 µm. As such the present biopolymers and specifically the alginate distinguishes itself over prior art biobased polymers, which are typically gel-like.

In an exemplary embodiment of the present product the super-absorbing biobased polymer comprises as the monovalent cation NH₄⁺ or Na⁺, and/or wherein the ALE is bleached. The monovalent cations may be a residue of pre-processing of the alginate. In addition the present biopolymers, being of biological origin, may require bleaching or the like.

In an exemplary embodiment of the present product the super-absorbing biobased polymer comprises 10⁻⁵-10⁻¹ wt.% of a water-miscible non-solvent, preferably 10⁻⁴-10⁻² wt.% non-solvent, such as mono- or dialkyl ketones, such as acetone, and alkoxy alkanols, such as iso propoxy ethanol, alkylene amines, such as alkylene mono-, di- or tri-amines. The water miscible non-solvent may be a residue of pre-treatment of the biopolymer.

In an exemplary embodiment the present product has an absorbing capacity of > 20 (wt./wt.), such as for an aqueous solution, typically > 25 (wt./wt.), and often > 30 (wt./wt.). As such the absorbing capacity is indeed superior and comparable or better than chemically obtained polymers or materials.

In an exemplary embodiment the present product further comprises a chemical compound selected from medicaments, such as antibiotics, anti-inflammatory agents, soil nutrients, such as N-, P- and K-comprising compounds and micro-nutrients, such as ammonia, urea, and ammonium nitrate, and combinations thereof. By upgrading the present product further functionality can be added.

In an exemplary embodiment of the present product the biobased polymer has a core-shell structure. Therewith the polymer can be protected from external influences and may also withstand mechanical forces better.

In an exemplary embodiment of the present product in the product the fibrous biobased polymer is at least one of spun, weaved, felted, needled, and punched. Therewith the present biobased polymer can be transformed such that is performs better for a given use of the product.

In an exemplary embodiment of the present product the product comprises a clay, such as an insulation material.

In an exemplary embodiment of the present product the product comprises a fire-retardant, such as a structural foam. Likewise the present SAP or solution thereof can be used as a cover or impregnation for preventing fire.

In an exemplary embodiment of the present product the product comprises UV-inhibitors.

In a second aspect the present invention relates to a method of obtaining a product according to the invention comprising providing a biopolymer, such as alginate, extracting the biopolymer with a water-miscible non-solvent forming a fibrous biopolymer, optionally cross-linking the fibrous biopolymer, e.g. by addition of a cross-linking agent, as defined in claim 12.

In an exemplary embodiment of the present method the cross-linking agent is selected from multivalent ions, such as Ca²⁺, Fe²⁺, Al³⁺, Mg²⁺, and Fe³⁺, nanoparticles, such as nano-CaCO₃, graphite, or wherein cross-linking is obtained by radiation.

In an exemplary embodiment the present method further comprises at least one of removing non-solvent, drying, freeze drying, and super-critical extraction, such as with CO₂.

The present invention also relates to an alternative method of obtaining the present comprising providing a biopolymer, such as alginate, forming a network gel, and forming a foam, such as by freeze-drying, and super-critical extraction, such as with CO₂, as defined in claim 14

In an exemplary embodiment of the present method the biopolymer comprises 0.1-80 wt.% clay, preferably 0.5-50 wt.%, more preferably 1-30 wt.%, such as 10-20 wt.%, wherein the clay may be natural clay or artificial clay, such as mont-morrilonite.

In an exemplary embodiment of the present method the network gel is formed by addition of multivalent ions, such as Ca2+, Fe2+, Al3+, Mg2+, and Fe3+, nanoparticles, such as nano-CaCO3, graphite, or wherein cross-linking is obtained by radiation, such as with CaCO3.

In a further alternative method granules of e.g. ALE may be directly obtained from granular sludge, such as in applications for slow release/uptake and suboptimal retention. Additional steps may be required such as sterilisation, retaining the granules in a designed mix of cations to get an optimal stiffness and strength, sterilisation/bleaching with peroxide or xCLO (Ca/Na hypochlorite bleach).

It has been found that the above methods are versatile in that by adapting parameters or using alternative steps SAP-properties, structure, and density can be changed.

The invention is further detailed by the accompanying figures and examples, which are exemplary and explanatory of nature and are not limiting the scope of the invention. To the person skilled in the art it may be clear that many variants, being obvious or not, may be conceivable falling within the scope of protection, defined by the present claims.

### SUMMARY OF THE FIGURES

Fig. 1 shows two photos of fibrous alginate obtained by the present process.
Fig. 2 shows a photo of foamed ALE.
Fig. 3 shows a photo of granular ALE.
Figs. 4a-d show SEM-photos of the present alginate.
Fig. 5a,b show a SEM-photo of a foamed alginate.
Fig. 6a-b show the present absorbing capacity (30 g/g).

### DETAILED DESCRIPTION OF THE FIGURES

Figure 1 shows a cotton-wool like example of the present fibrous alginate after freeze drying. The length is a few centimeters. First (i) a 3% Na-alginate solution in H2O was prepared, (ii) the solution was left to homogenise and some occasional stirring was applied, (iii) thereafter about 50% acetone to the alginate/H₂O solution was added, decant on top and shaken vigorously, (iv) then the supernatant was poured off and replaced with 100% acetone. This was repeated several times. A fibrillar mass of Na-alginate in acetone with a low water content was obtained. Then (v) the moistened fibrillar mass was placed in a freezer and then the solvent was evaporated off in vacuum; thereby a freeze dried Na-alginate fibrillar mass was obtained. The freeze drying procedure is found to prevent the sample from collapsing so the morphology can be investigated more easily. Overall sample size is about 4 cm long, 1 cm diameter.

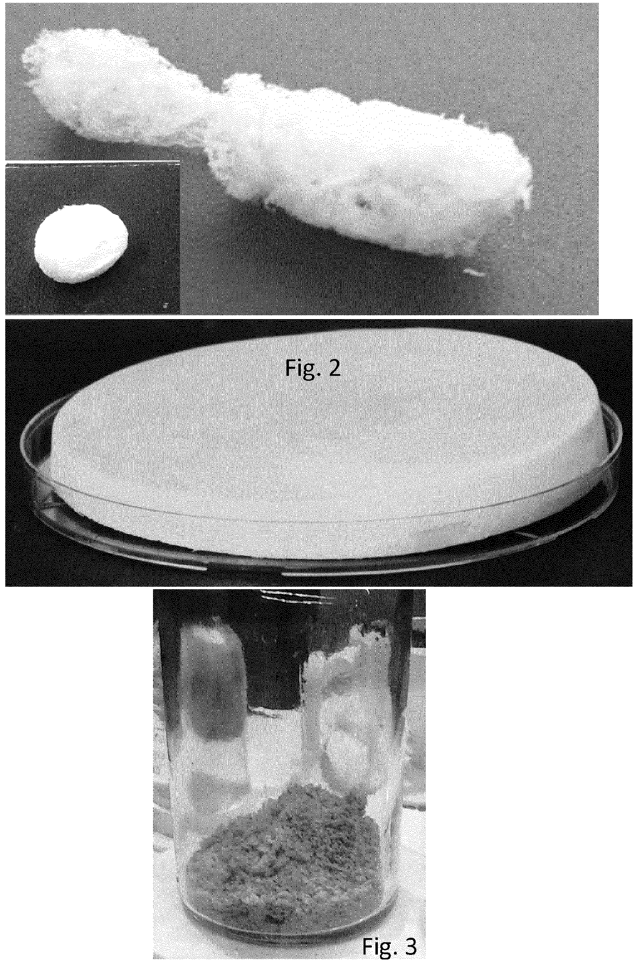

Fig. 2 shows a photo of foamed ALE.

Fig. 3 shows a photo of granular ALE.

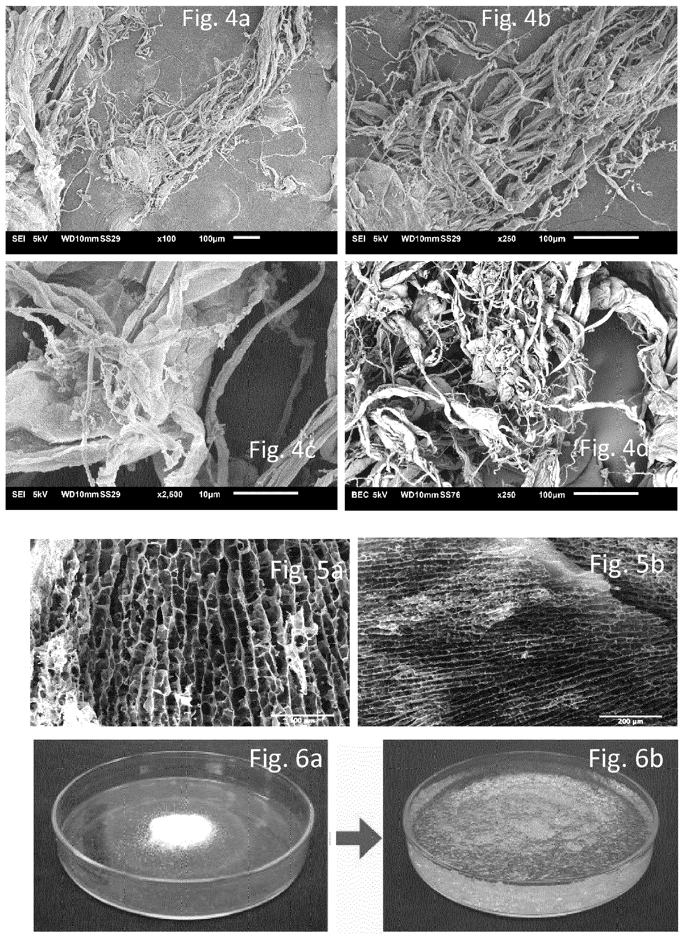

Figures 4a-d show SEM photos of the present Na-alginate. Typical fibres obtained are a few (1-2) mm to a few (1-5) cm long having a thickness of 2-250 µm. Small fragments were imaged using a SEM (see scale bars for magnification and SEM settings). Various techniques were used, such as sputtered with Au to prevent charging, and also a backscatter image.

Fig. 5a,b show SEM-photos of a foamed alginate.

### EXAMPLES/EXPERIMENTS

The invention although described in detailed explanatory context may be best understood in conjunction with the accompanying examples.

### Diaper comprising ALE

A diaper, and likewise a sanitary towel or incontinence pad, is made with a 2-ply absorber. Therein a layer for liquid up-take, distribution and intermediate storage layer on the body side in use is provided and a storage layer containing >= 50 wt.% super absorbing polymer (ALE) on the other side. The first layer may consist of a thermoplastic polymer, extruded with addition of a blowing agent. In addition a back-side layer may be provided, preferably a liquid impermeable backsheet. The SAP preferably has a pH<6, such as less than 5.0. In addition additives may be provided, such as absorbents, such as benzoic acid, hydroxybenzoic acid, and esters thereof, which are found to suppress unpleasant odours. In addition to the above materials the absorbent core may comprise, e.g. in admixture, other absorbent materials. Any other suitable absorbent material can be used. Examples include a wide variety of liquid-absorbent materials commonly used in disposable diapers and other absorbent articles such as cellulose based materials, tissues, laminates, foams, sponges, gelling materials, and combinations of materials. Also further fibre materials may be included of natural and synthetic origin, such as cotton. To determine a fluid retention capacity of the super-absorbent material in saline after centrifugation, the standard test EDANA WSP 241.2 is used.

### Wound dressing

An example of an alginate antibacterial dressing comprises: 50-90% of super absorbing alginate, 5-50% of beta-cyclodextrin and 0.0-5% of silver ion dispersion liquid. The alginate material supports hydrogen-bond action in molecules.

### Soil improver

An example of a soil improver composition 50% ALE is provided therein. The improver may further comprise 2-60% of straw, 1-50% humus, and 1-10% of inorganic salts; the inorganic salts may comprise calcium, magnesium, iron, zinc, boron and nitrogen. The improver is quickly effective and durable; the improver need only be applied for 1 to 2 times. The ALE enhances the permeability of the soil and reduces soil corrosion resistance. It further has a good water retention, and shows slow-release performance for fertilizers. The soil improver may find use as a stabilizer, as fillings, for improving physical-chemical properties, increasing its cementing value, and decreasing its linear expansion and contraction. The present soil improver is especially suited for soils in dry climates (semi-arid and arid) and in continental climates. It is found to reduce water consumption of up to 50%, to limit fertilizer leaching and to improve soil.

### Water retainer

An example of a cold retainer composition is 25% ALE, 5% urea, a polyhydric alcohol, and water. The cold retainer composition has an excellent durability of cold retaining effect, can be folded to an applied part and provides softness at a low temperature; it may be used for cooling of strains, injuries in general, fractions, ligaments of the body, etc.

### Absorbent pan or pillow

The present SAPS can be used in absorbent pans, drip pans, and pillows, for absorbing liquids in general, or spills thereof, such as for use under machines or hydraulic hoses. Liquids may be coolants, solvents, water, and urine. They typically comprise 20-50 wt.% of SAP. A typical capacity may be a few litres per pan/pillow, up to about 100 litre. The pan or pillow may be provided with a resistant material, such as poly propylene.

### Recipe for forming foamed alginate/montmorillonite

A 3 wt.% Na-Alginate solution was prepared and also a completely exfoliated 3 wt.% Na MMT suspension was prepared. These were mixed together in a 50:50 ratio to obtain a homogenous suspension. Following that a slow release (low solubility) Ca²⁺-based salt was mixed, for example a dicalcium silicate. In alternative approaches CaCO₃, Ca-citrate, and Ca-oxalate were used. The final concentration of the Na-Alginate:Na-MMT:Ca₂SiO₄ was 49:49:2. The system was found to gel internally via Ca cross linking and it formed a hydrogel nanocomposite, which was freeze dried with liquid nitrogen during 2 days at a temperature of about -200 °C at a pressure of about 10 Pa to obtain the present high porosity, lightweight foam structure.

### Swelling performance

| Swelling g/g | BASF Luquasorb | Pamper granules | Sodium ALE | Ca alginate |
|---|---|---|---|---|
| Density | 0.7 | 0.271 | 0.0041 | 0.0035 |
| Demi free | 384 | 270 | 19.4 | 21.38 |
| Saline free | 50 | 46.6 | 19.3 | 16.0 |
| AUL (1.0 Psi) | >17 | 18.8 | 10.1 | 5.2 |

The density of the present SAPs (column 4 and 5) is much lower than that of comparable commercial SAPs. The swelling properties are somewhat lower, but for higher saline liquids are comparable.

## Claims

1. Product for liquid absorption comprising
a super-absorbing material of an ionic biobased polymer, wherein the biobased polymer is produced by a microorganism selected from bacteria, comprising 20-99.8 wt.% low density polymer material, with a density <0.5 gr/cm³,
wherein the super-absorbing biobased polymer material is a non-woven material, and/or wherein the super-absorbing biobased polymer material comprises fibres, and/or wherein the wherein the super-absorbing biobased polymer material comprises a powder with particle sizes > 0.1 mm,
0-80 wt.% fillers,
0-10 wt.% cross-linking agent,
0-30 wt.% water-soluble polymer,
0-2 wt.% buffer,
0-2 wt.% plasticizer,
0-5 wt.% lipids, and
the remainder being water, wherein all percentages are relative to a total weight of the super-absorbing material.

2. Product according to claim 1, wherein the product is selected from a diaper, an incontinence product, concrete, a personal hygiene product, a soil-improver, a water retainer, a wound dressing, especially for burn wounds, a liquid absorbent, a thermal insulator, a thermal clothing layer, a thermal insulating panel, a protective sheet or laminate, such as a cable wrap or tape, a filter, such as a gas filter, a particle filter, and a liquid filter.

3. Product according to any of claims 1-2, wherein the biobased polymer is produced by a microorganism, selected from the order Pseudomonadaceae, such as *pseudomonas* or *Acetobacter bacteria*, the order Planctomycetales, such as *Brocadia anammoxidans, Kuenenia stuttgartiensis*, or *Brocadia fulgida*, and algae, such as *brown algae*, and the biopolymer is bacterial alginate, and/or
wherein the biopolymer comprises a polysaccharide.

4. Product according to any of claims 1-3, wherein the super-absorbing biobased polymer is immobilized in the product, such as by at least one of pressing, gluing, embedding in a textile or non-woven garment, attaching, stitching, weaving, felting, and needling.

5. Product according to any of claims 1-4, wherein the super-absorbing biobased polymer is treated by at least one of cross-linking, such as with multivalent ions, such as Ca²⁺, Fe²⁺, Al³⁺, Mg²⁺, and Fe³⁺, with nanoparticles, such as nano-CaCO₃, and graphite, by radiation branching, by chemical modification, such as for preventing dissolving, for preventing adhesion, by physical modification, such as changing a fibre cross-section and/or fibre length, by surface modification, such as changing hydrophobicity and hydrophilicity, and by providing a shell.

6. Product according to any of claims 1-5, wherein the super-absorbing biobased polymer comprises fibres with a diameter of 1-100 µm, and a length of 0.1-5 mm, and/or
wherein the biobased polymer has a core-shell structure, and/or
wherein the fibrous biobased polymer is at least one of spun, weaved, felted, and needled.

7. Product according to any of claims 1-6, wherein the super-absorbing biobased polymer comprises as monovalent cation NH₄⁺ or Na⁺, and/or wherein the bacterial alginate is bleached.

8. Product according to any of claims 1-7, wherein the super-absorbing biobased polymer comprises 10⁻⁵-10⁻¹ wt.% of a water-miscible biopolymer non-solvent, preferably 10⁻⁴-10⁻² wt.% non-solvent, such as mono- or dialkyl ketones, such as acetone, and alkoxy alkanols, such as iso propoxy ethanol, and alkylene amines, such as alkylene mono-, di- or tri-amines.

9. Product according to any of claims 1-8, further comprising a chemical compound selected from medicaments, such as antibiotics, anti-inflammatory agents,
soil nutrients, such as N-, P- and K-comprising compounds and micro-nutrients, such as ammonia, urea, and ammonium nitrate, and combinations thereof.

10. Product according to any of claims 1-9, wherein the product comprises multiple layers, of which one layer comprises the SAP-material.

11. Product according to any of claims 1-10, wherein the product comprises a clay, such as an insulation material, and/or wherein the product comprises a fire-retardant, such as a structural foam, and/or
wherein the product comprises UV-inhibitors.

12. Method of obtaining a super-absorbing biobased polymer material, wherein the super-absorbing biobased polymer material comprises fibres, for a product according to any of claims 1-11, comprising
providing an ionic biopolymer, wherein the biobased polymer is produced by a microorganism, in particular bacterial alginate,
extracting the biopolymer with a water-miscible biopolymer non-solvent, forming a fibrous biopolymer, and optionally cross-linking the fibrous biopolymer, such as by addition of a cross-linking agent,
preferably wherein the cross-linking agent is selected from multivalent ions, such as Ca²⁺, Fe²⁺, Al³⁺, Mg²⁺, and Fe³⁺, graphite, or wherein cross-linking is obtained by radiation.

13. Method according to claim 12, further comprising at least one of removing non-solvent, drying, freeze drying, and super-critical extraction, such as with CO₂.

14. Method of obtaining a super-absorbing biobased polymer material, wherein the super-absorbing biobased polymer material comprises a powder, for a product according to any of claims 1-11, comprising
providing an ionic biopolymer, wherein the biobased polymer is produced by a microorganism, such as bacterial alginate,
forming an internal network gel, and
forming a foam, such as by freeze-drying, and super-critical extraction, such as with CO₂, and
forming the powder thereof with particle sizes > 0.1 mm, preferably wherein the biopolymer comprises 0.1-80 wt.% clay, preferably wherein the internal network gel is formed by addition of multivalent ions, such as Ca²⁺, Fe²⁺, Al³⁺, Mg²⁺, and Fe³⁺, such as with CaCO₃, graphite, or wherein cross-linking is obtained by radiation.

## Patentansprüche

1. Produkt zur Flüssigkeitsabsorption, umfassend
ein superabsorbierendes Material aus einem ionischen biobasierten Polymer, wobei das biobasierte Polymer durch einen Mikroorganismus, ausgewählt aus Bakterien, hergestellt wird, das 20-99,8 Gew.-% Polymermaterial mit niedriger Dichte umfasst, mit einer Dichte <0,5 gr/cm³,
wobei das superabsorbierende biobasierte Polymermaterial ein Vliesmaterial ist, und/oder wobei das superabsorbierende biobasierte Polymermaterial Fasern umfasst, und/oder wobei das superabsorbierende biobasierte Polymermaterial ein Pulver mit Partikelgrößen > 0,1 mm umfasst,
0-80 Gew.-% Füllstoffe,
0-10 Gew.-% Vernetzungsmittel,
0-30 Gew.-% wasserlösliches Polymer,
0-2 Gew.-% Puffer,
0-2 Gew.-% Weichmacher,
0-5 Gew.-% Lipide, und
ein Rest Wasser, wobei sich alle Prozentangaben auf das Gesamtgewicht des superabsorbierenden Materials beziehen.

2. Produkt nach Anspruch 1, wobei das Produkt ausgewählt ist aus einer Windel, einem Inkontinenzprodukt, Beton, einem Körperpflegeprodukt, einem Schmutzverbesserer, einem Wasserrückhaltemittel, einem Wundverband, insbesondere für Verbrennungswunden, einem flüssigkeitsabsorbierenden Mittel, einem Wärmeisolator, einer thermischen Bekleidungsschicht, einer wärmeisolierenden Platte, einer Schutzfolie oder einem Schutzlaminat, wie zum Beispiel einer Kabelumwicklung oder einem Klebeband, einem Filter, wie zum Beispiel einem Gasfilter, einem Partikelfilter und einem Flüssigkeitsfilter, ausgewählt wird.

3. Produkt nach einem der Ansprüche 1-2, wobei das biobasierte Polymer von einem Mikroorganismus, ausgewählt aus der Ordnung *Pseudomonadaceae*, wie *Pseudomonas-* oder *Acetobacter*-Bakterien, der Ordnung *Planctomycetales*, wie *Brocadia anammoxidans*, *Kuenenia stuttgartiensis* oder *Brocadia fulgida*, und Algen, wie Braunalgen, hergestellt wird und das Biopolymer bakterielles Alginat ist, und/oder
wobei das Biopolymer ein Polysaccharid umfasst.

4. Produkt nach einem der Ansprüche 1-3, wobei das superabsorbierende biobasierte Polymer in dem Produkt immobilisiert ist, wie zum Beispiel durch mindestens eines der folgenden Verfahren: Pressen, Kleben, Einbetten in ein textiles oder nicht-gewebtes Kleidungsstück, Anbringen, Nähen, Weben, Filzen, und Nadeln.

5. Produkt nach einem der Ansprüche 1-4, wobei das superabsorbierende biobasierte Polymer durch mindestens eines der folgenden Verfahren behandelt wird: Vernetzung, beispielsweise mit mehrwertigen Ionen, wie Ca²⁺, Fe²⁺, Al³⁺, Mg²⁺, und Fe³⁺,mit Nanopartikeln, wie Nano-CaCO₃, und Graphit, durch Strahlenverzweigung, durch chemische Modifikation, zum Beispiel zur Verhinderung der Auflösung, zur Verhinderung der Adhäsion, durch physikalische Modifikation, zum Beispiel durch Veränderung des Faserquerschnitts und/oder der Faserlänge, durch Oberflächenmodifikation, zum Beispiel durch Veränderung der Hydrophobie und Hydrophilie, und durch Bereitstellung einer Hülle.

6. Produkt nach einem der Ansprüche 1-5, wobei das superabsorbierende biobasierte Polymer Fasern mit einem Durchmesser von 1-100 µm und einer Länge von 0,1-5 mm umfasst, und/oder
wobei das biobasierte Polymer eine Kern-Schale-Struktur aufweist, und/oder
wobei das faserige biobasierte Polymer mindestens eine der folgenden Eigenschaften aufweist: gesponnen, gewebt, verfilzt und genadelt.

7. Produkt nach einem der Ansprüche 1-6, wobei das superabsorbierende biobasierte Polymer ein einwertiges Kation NH₄⁺ oder Na⁺ enthält und/oder wobei das bakterielle Alginat gebleicht ist.

8. Produkt nach einem der Ansprüche 1-7, wobei das selbstabsorbierende biobasierte Polymer 10⁻⁵-10⁻¹ Gew.-% eines mit Wasser mischbaren Biopolymer-Nichtlösungsmittels, vorzugsweise 10⁻⁴-10⁻² Gew.-% Nichtlösungsmittel, wie Mono- oder Dialkylketone, wie Aceton, und Alkoxyalkanole, wie Isopropoxyethanol, und Alkylenamine, wie Alkylenmono-, -di- oder -triamine, umfasst.

9. Produkt nach einem der Ansprüche 1-8, das ferner eine chemische Verbindung enthält, die ausgewählt ist aus Arzneimitteln, wie Antibiotika, entzündungshemmenden Mitteln, Bodennährstoffen, wie N-, P- und K-haltigen Verbindungen und Mikronährstoffen, wie Ammoniak, Harnstoff und Ammoniumnitrat, und Kombinationen davon.

10. Produkt nach einem der Ansprüche 1-9, wobei das Produkt mehrere Schichten umfasst, von denen eine Schicht das SAP-Material enthält.

11. Erzeugnis nach einem der Ansprüche 1-10, wobei das Erzeugnis einen Ton, wie zum Beispiel ein Isoliermaterial, enthält, und/oder
wobei das Produkt ein feuerhemmendes Mittel, wie zum Beispiel einen Strukturschaum, enthält, und/oder
wobei das Produkt UV-Inhibitoren enthält.

12. Verfahren zur Gewinnung eines superabsorbierenden biobasierten Polymermaterials, wobei das superabsorbierende biobasierte Polymermaterial Fasern umfasst, für ein Produkt nach einem der Ansprüche 1-11, umfassend
Bereitstellen eines ionischen Biopolymers, wobei das biobasierte Polymer durch einen Mikroorganismus, insbesondere bakterielles Alginat, hergestellt wird,
Extrahieren des Biopolymers mit einem mit Wasser mischbaren Biopolymer-Nichtlösungsmittel, wobei ein faseriges Biopolymer gebildet wird, und
gegebenenfalls Vernetzen des faserigen Biopolymers, beispielsweise durch Zugabe eines Vernetzungsmittels,
wobei das Vernetzungsmittel vorzugsweise ausgewählt ist aus mehrwertigen Ionen, wie Ca²⁺, Fe²⁺, Al³⁺, Mg²⁺, und Fe³⁺, Graphit, oder wobei die Vernetzung durch Bestrahlung erreicht wird.

13. Verfahren nach Anspruch 12, das ferner mindestens eines der folgenden Verfahren umfasst:
Entfernen von Nicht-Lösungsmittel, Trocknen, Gefriertrocknen und superkritische Extraktion, beispielsweise mit CO₂.

14. Verfahren zur Gewinnung eines superabsorbierenden biobasierten Polymermaterials, wobei das superabsorbierende biobasierte Polymermaterial ein Pulver umfasst, für ein Produkt nach einem der Ansprüche 1-11, umfassend
Bereitstellen eines ionischen Biopolymers, wobei das biobasierte Polymer von einem Mikroorganismus, wie bakteriellem Alginat, hergestellt wird,
Bilden eines internen Netzwerkgels, und
Bildung eines Schaums, beispielsweise durch Gefriertrocknung, und
überkritische Extraktion, zum Beispiel mit CO₂, und
Bildung des Pulvers mit Partikelgrößen > 0,1 mm,
wobei das Biopolymer vorzugsweise 0,1-80 Gew.-% Ton enthält,
wobei das interne Netzwerkgel vorzugsweise durch Zugabe von mehrwertigen Ionen, wie Ca²⁺, Fe²⁺, Al³⁺, Mg²⁺, und Fe³⁺,wie mit CaCO₃, Graphit, gebildet wird oder wobei die Vernetzung durch Bestrahlung erreicht wird.

## Revendications

1. Produit pour l'absorption de liquides comprenant
un matériau super absorbant d'un polymère biosourcé ionique, dans lequel le polymère biosourcé est produit par un microorganisme choisi parmi les bactéries, comprenant 20-99,8 % en poids de matériau polymère de faible densité, avec une densité <0,5 g/cm³,
dans lequel le matériau polymère biosourcé super absorbant est un matériau non tissé, et/ou dans lequel le matériau polymère biosourcé super absorbant comprend des fibres, et/ou dans lequel le matériau polymère biosourcé super absorbant comprend une poudre avec des tailles de particules > 0,1 mm,
0-80 % en poids de charges,
0-10 % en poids d'agent de réticulation,
0-30% en poids de polymère hydrosoluble,
0-2 % en poids de tampon,
0-2 % en poids de plastifiant,
0-5 % en poids de lipides, et
le reste étant de l'eau, tous les pourcentages étant rapportés au poids total du matériau super-absorbant.

2. Produit selon la revendication 1, dans lequel le produit est choisi parmi une couche, un produit d'incontinence, du béton, un produit d'hygiène personnelle, un produit d'amélioration des sols, un produit de rétention d'eau, un pansement, en particulier pour les brûlures, un absorbant de liquide, un isolant thermique, une couche de vêtements thermiques, un panneau d'isolation thermique, une feuille ou un stratifié de protection, tel qu'une enveloppe ou une bande de câble, un filtre, tel qu'un filtre à gaz, un filtre à particules, et un filtre à liquide.

3. Produit selon l'une quelconque des revendications 1-2, dans lequel le polymère biosourcé est produit par un micro-organisme, choisi parmi l'ordre des *Pseudomonadaceae*, comme les bactéries *Pseudomonas* ou *Acetobacter*, l'ordre des *Planctomycetales*, comme *Brocadia anammoxidans*, *Kuenenia stuttgartiensis*, ou *Brocadia fulgida*, et les algues, comme les algues brunes, et le biopolymère est l'alginate bacterial, et/ou
dans lequel le biopolymère comprend un polysaccharide.

4. Produit selon l'une quelconque des revendications 1-3, dans lequel le polymère biosourcé super absorbant est immobilisé dans le produit, tel que par au moins l'un des procédés suivants : pressage, collage, incorporation dans un vêtement textile ou non tissé, fixation, couture, tissage, feutrage, et aiguilletage.

5. Produit selon l'une quelconque des revendications 1-4, dans lequel le polymère biosourcé super absorbant est traité par au moins l'un des moyens suivants : réticulation, par exemple avec des ions multivalents, tels que Ca²⁺, Fe²⁺, Al³⁺, Mg²⁺, et Fe³⁺, avec des nanoparticules, telles que du nano-CaCO₃, et du graphite, par ramification par rayonnement, par modification chimique, par exemple pour empêcher la dissolution, pour empêcher l'adhésion, par modification physique, par exemple pour changer la section transversale et/ou la longueur de la fibre, par modification de la surface, par exemple pour changer l'hydrophobie et l'hydrophilie, et en fournissant une enveloppe.

6. Produit selon l'une quelconque des revendications 1-5, dans lequel le polymère biosourcé super absorbant comprend des fibres d'un diamètre de 1 à 100 µm, et d'une longueur de 0,1 à 5 mm, et/ou dans lequel le polymère biosourcé a une structure cœur-coquille, et/ou
dans lequel le polymère biosourcé fibreux est au moins l'un des éléments suivants: filé, tissé, feutré et aiguilleté.

7. Produit selon l'une quelconque des revendications 1-6, dans lequel le polymère biosourcé super absorbant comprend comme cation monovalente NH₄⁺ ou Na⁺, et/ou dans lequel l'alginate bactérien est blanchi.

8. Produit selon l'une quelconque des revendications 1-7, dans lequel le polymère biosourcé super absorbant comprend 10⁻⁵-10⁻¹ % en poids d'un non-solvant biopolymère miscible à l'eau, de préférence 10⁻⁴-10⁻² % en poids de non-solvant, tel que des mono- ou dialkylcétones, telles que l'acétone, et des alcoxyalcanols, tels que l'isopropoxyéthanol, et des alkylèneamines, telles que des alkylènemono-, di- ou tri-aminés.

9. Produit selon l'une quelconque des revendications 1-8, comprenant en outre un composé chimique choisi parmi les médicaments, tels que les antibiotiques, les agents anti-inflammatoires,
les nutriments du sol, tels que les composés comprenant N-, P- et K et les micro-nutriments, tels que l'ammoniac, l'urée et le ni-trate d'ammonium, et leurs combinaisons.

10. Produit selon l'une quelconque des revendications 1-9, dans lequel le produit comprend des couches multiples, dont une couche comprend le matériau SAP.

11. Produit selon l'une quelconque des revendications 1-10, dans lequel le produit comprend une argile, telle qu'un matériau d'isolation, et/ou
dans lequel le produit comprend un retardateur de feu, tel qu'une mousse structurelle, et/ou dans lequel le produit comprend des inhibiteurs d'UV.

12. Procédé d'obtention d'un matériau polymère biosourcé super absorbant, dans lequel le matériau polymère biosourcé super absorbant comprend des fibres, pour un produit selon l'une quelconque des revendications 1 à 11, comprenant
La fourniture d'un biopolymère ionique, dans lequel le polymère biosourcé est produit par un micro-organisme, en particulier un alginate bactérien,
l'extraction du biopolymère avec un non-solvant biopolymère miscible à l'eau, formant un biopolymère fibreux, et
éventuellement réticuler le biopolymère fibreux, par exemple par l'addition d'un agent de réticulation, de préférence dans lequel l'agent de réticulation est choisi parmi les ions multivalents, tels que Ca²⁺, Fe²⁺, Al³⁺, Mg²⁺, et Fe³⁺, le graphite, ou dans lequel la réticulation est obtenue par rayonnement.

13. Procédé selon la revendication 12, comprenant en outre au moins l'un parmi l'élimination du non-solvant, le séchage, la lyophilisation, et l'extraction supercritique, telle que par CO₂.

14. Procédé d'obtention d'un matériau polymère biosourcé super absorbant, dans lequel le matériau polymère biosourcé super absorbant comprend une poudre, pour un produit selon l'une quelconque des revendications 1 à 11, comprenant les étapes suivantes
fournir un biopolymère ionique, dans lequel le polymère biosourcé est produit par un micro-organisme, tel qu'un alginate bactérien,
la formation d'un gel à réseau interne, et
la formation d'une mousse, par exemple par lyophilisation, et
une extraction supercritique, par exemple avec du CO₂, et
la formation de sa poudre avec des tailles de particules > 0,1 mm,
de préférence dans lequel le biopolymère comprend 0,1-80 % en poids d'argile, de préférence dans lequel le gel de réseau interne est formé par l'addition d'ions multivalents, tels que Ca²⁺, Fe²⁺, Al³⁺, Mg²⁺, et Fe³⁺, comme avec CaCO₃, graphite, ou dans lequel la réticulation est obtenue par radiation.
